# EUROPÄISCHE PATENTANMELDUNG

(11) **EP 3 620 781 A1**
(43) Veröffentlichungstag der Anmeldung: **11.03.2020**
(21) Anmeldenummer: 19188226.5
(22) Anmeldetag: 25.07.2019
(51) Int. Cl.: G01N 27/04, G01N 27/20, B66B 7/12

(54) **VERFAHREN ZUR DETEKTION EINES ABNUTZUNGSZUSTANDS EINES TRAGRIEMENS**

(30) Priorität: 28.08.2018 DE 102018214515
(71) Anmelder: ContiTech Antriebssysteme GmbH, 30165 Hannover (DE)
(72) Erfinder: Göser, Hubert, 30165 Hannover (DE); Quass, Jan-Henning, 30165 Hannover (DE); Reck, Siegfried, 30165 Hannover (DE)
(74) Vertreter: Kilsch, Armin Ralph

(57) **Zusammenfassung**

Verfahren zur Detektion eines Abnutzungszustands eines Tragriemens aus elastomerem Material innerhalb eines Trag- bzw. Aufzugsystems mit Hilfe der Zeitbereichsreflektometrie, wobei die Tragriemen elektrisch leitfähige Zugträger aufweisen und jeweils ein erster und ein zweiter Zugträger als Wellenleiter innerhalb einer Mess-Elektronik verschaltet werden und die Wellenleiter mit Hilfe eines Widerstands abgeschlossen werden, dessen Widerstandswert Zo gleich groß ist wie der Leitungswellenwiderstand zum Zeitpunkt der Inbetriebnahme eines neu aufgelegten Tragriemens, wobei an einem ersten Ende des Tragriemens durch einen Signalgenerator ein Signalimpuls auf den ersten der jeweils als Wellenleiter geschalteten zwei Zugträger aufgegeben bzw. eingekoppelt und über eine Auswerteelektronik dann ermittelt wird, ob nach Einkopplung des Signalimpulses eine Reflexion des Signalimpulses vorhanden ist und welche Amplituden und Zeitverläufe die reflektierten Signalimpulse aufweisen. Bei einer Reflexion eines Signalimpulses wird anhand der Signallaufzeiten der Abstand und die Ausprägung der die Reflexion erzeugenden Stelle ermittelt und damit auf die Ablegereife des Tragriemens geschlossen.

## Beschreibung

Die Erfindung betrifft ein Verfahren zur Detektion eines Abnutzungszustands eines Tragriemens aus elastomerem Material innerhalb eines Trag- bzw. Aufzugsystems mit Hilfe der Zeitbereichsreflektometrie, wobei die Tragriemen in ihrer Längsrichtung im Riemenkörper koaxial eingebettete elektrisch leitfähige Zugträger bzw. Verstärkungselemente aufweisen, die als aus mehreren Filamenten getwistete oder geschlagene Corde ausgebildet sind. Der Abnutzungszustand ist dabei nach Art und Umfang bestimmbar.

Weiterhin betrifft die Erfindung eine zur Durchführung des Verfahrens besonders geeignete Einrichtung sowie ein Aufzugsystem mit einer solchen Einrichtung.

Insbesondere bei Personen- oder Lastenaufzügen unterliegen die Tragmittel der Kabinen, üblicherweise Stahlseile oder Aufzugsgurten, bei denen Stahlseile oder Stahlcorde als Zugträger in einer Gummi- oder Kunststoffmatrix eingebettet sind, strengen Regelungen in Bezug auf ihre Betriebssicherheit und Zugfestigkeit. Die Zugfestigkeit kann sich im Laufe der Lebensdauer eines Aufzugs durch Setzungen, Materialverschleiß oder durch Ermüdungserscheinungen innerhalb des Tragmittels reduzieren. Prüfungsnormen geben hierzu entsprechende Regelungen vor. Aufzugsanlagen werden von entsprechenden Prüfungsorganisationen sorgfältig auf die Einhaltung dieser Reglungen und gesetzlichen Vorschriften überprüft.

Sobald ein Tragmittel die gesetzlichen Anforderungen nicht mehr erfüllt, muss es ausgetauscht, d.h. abgelegt werden, was bedeutet, dass es aus dem Aufzugssystem entfernt und durch ein neues Tragmittel ersetzt wird. Dieser Zeitpunkt ist die sogenannte "Ablegereife".

Das Erreichen der Ablegereife bei den im Stand der Technik für Aufzüge häufig verwendeten Stahlseilen unter anderem durch eine optische Kontrolle ermittelt. Hierzu kann bei Stahlseilen z.B. eine Beurteilung des Zustandes der Zugfestigkeit durch optische Kontrolle der einzelnen Litzen auf Brüche erfolgen oder durch Messung eines sich etwa verändernden Durchmessers eines Stahlseils. Solche Methoden sind in der Normung aufgenommen und gehören daher zum Stand der Technik.

Bei den heute mehr und mehr als Tragmittel verwendeten Aufzugsgurten, bei denen Stahlseile oder Stahlcorde als Zugträger in einer Gummimatrix eingebettet sind, ist eine solche Kontrolle mittels rein optischer Verfahren kaum möglich, so dass es zusätzlicher verfeinerter Messmethoden bedarf.

Hier nutzt man eine Kombination aus optischer und messtechnischer bzw. statistischer Methoden. In der Regel ist dabei zunächst die optische Integrität der Tragmittel zu überprüfen und sicherzustellen. Die Festigkeit von metallischen Zugelementen im inneren der Kunststoffmatrix kann z. B. mittels Widerstandsmessung erfolgen. Hierzu werden die einzelnen Zugträger elektrisch miteinander verbunden. Eine Querschnittsänderung der Zugträger infolge von Reibung (Fretting) oder l.itzenbrüchen verändern z.B. den Widerstand im elektrischen Leiter, was als Indikator für die Ablegereife herangezogen werden kann.

Die US 2004/0046540 A1 offenbart ein Verfahren, bei dem eine Abnutzung eines Seils oder auch eines Seile enthaltenden Aufzugsgurts mit einer Vielzahl von ferromagnetischen Litzen oder Corden dadurch überprüft werden kann, das die Veränderung der magnetischen Feldstärke bzw. Veränderungen im magnetischen Fluss diktiert werden. Eine solche Überprüfung kann durch laufend während des Betriebes erfolgen, weist jedoch den Nachteil auf, dass relativ komplizierte Vorrichtungen und Apparate zur Magnetisierung bzw. zur Ermittlung der Feldstärkenveränderung eingesetzt werden müssen.

Die EP 0 845 672 A1 offenbart ebenfalls ein magnetisches Prüfungsverfahren während des Betriebes eine Prüfung der magnetischen Eigenschaften eines magnetisierbaren länglichen Gegenstandes erlaubt, etwa eines Aufzuggurtes, bei dem Hall-Sensoren etwaige Durchmesserveränderungen der Zugträger anhand der Veränderungen der magnetischen Eigenschaften ermitteln. Auch diese Einrichtung erfordert einen relativ hohen konstruktiven Aufwand.

Zur Nutzung der Zeitbereichsreflektometrie, im Englischen als Time-Domain-Reflectometry (TDR) bekannt, gelegentlich auch als "Kabelradar" bezeichnet, sind bereits seit den 1960er Jahren Geräte bekannt, mit deren Hilfe Kabelbrüche und Quetschungen in langen Elektrokabeln mehr oder weniger präzise geortet werden konnten. Dabei erzeugt ein Impulsgenerator eine Folge elektrischen Impulsen, die auf den Innenleiter eines Koaxialkabels aufgegeben werden. Mit Hilfe eines an beide Kabelenden angeschlossenen Oszilloskops können die elektrischen Eigenschaften des Kabels überprüft werden. Eine Anpassung des Kabelradars im Hinblick auf andere Anwendungen, beispielsweise bei Stahlseilen in Aufzugsystemen, ist jedoch aufgrund der dortigen konstruktiven Besonderheiten nie in Betracht gezogen worden.

Für die Erfindung bestand also die Aufgabe, ein Verfahren bereitzustellen, mit dem eine sichere Bestimmung des Abnutzungszustands von Zug- oder Tragmitteln in Form von mit Zugträgern verstärkten Tragriemen, insbesondere von Aufzugsriemen möglich wird und welches nur einen geringen apparativen/konstruktiven Aufwand für die dazugehörigen Vorrichtungen beinhaltet.

Gelöst wird diese Aufgabe durch die Merkmale des Hauptanspruchs. Weitere vorteilhafte Ausbildungen sind in den Unteransprüchen offenbart.

Dabei werden jeweils ein erster und ein zweiter Zugträger des Tragriemens als Wellenleiter innerhalb einer Mess-Elektronik verschaltet, also in Form einer Messschaltung verwendet. Bei den üblicherweise mehreren parallel angeordneten Zugträgern eines Tragriemens können ein solches Paar von Zugträgern stellvertretend für die übrigen verwendet oder aber nach eine zufälligen oder vorgegebenen Routine alle Zugträger beliebig abwechselnd zu Wellenleitern verbunden werden.

Die jeweils als Wellenleiter geschalteten zwei Zugträger werden mit Hilfe eines aus mehreren Einzelimpedanzen aufgebauten Netzwerks an einem zweiten Ende des Tragriemens über einen Widerstand abgeschlossen bzw. miteinander verbunden. Dessen Widerstandswert Z₀ ist gleich groß wie der Leitungswellenwiderstand (Wellenimpedanz), der zum Zeitpunkt der Inbetriebnahme eines neu aufgelegten Tragriemens bei der Verschaltung zweier Zugträger zu einem Wellenleiters vorhanden ist.

An einem ersten Ende des Tragriemens wird durch einen Signalgenerator ein Signalimpuls auf den ersten der jeweils als Wellenleiter geschalteten zwei Zugträger aufgegeben bzw. eingekoppelt. Über eine Auswerteelektronik vorzugsweise am ersten Ende des Tragriemens wird dann ermittelt, ob nach Einkopplung des Signalimpulses eine Reflexion des Signalimpulses vorhanden ist und welche Amplituden und Zeitverläufe die reflektierten Signalimpulse aufweisen,

Bei einer detektierten Reflexion eines Signalimpulses wird anhand der Signallaufzeiten der Abstand der die Reflexion erzeugenden Stelle von der Einkoppelstelle am ersten Ende berechnet. Die Ausprägung der die Reflexion erzeugenden Stelle im Tragriemen anhand eines vorgegebenen Katalogs wird ermittelt und abhängig davon auf die Ablegereife des Tragriemens geschlossen.

Dadurch, dass aus zwei Zugträgern ausgebildete Wellenleiter an seinem zweiten Ende, das entspricht dem zweiten Ende des Tragriemens, über den genannten Widerstand mit dem Widerstandswert Z₀ abgeschlossen wird, der gleich groß ist wie der Leitungswellenwiderstand (Wellenimpedanz) eines aus neuen, ungebrauchten Zugträgern bestehenden Wellenleiters, also dem zum Zeitpunkt der Inbetriebnahme eines neu aufgelegten Tragriemens vorhandenen Wellenwiderstands entspricht, werden die eingekoppelten elektrischen Impulse am Ende des Wellenleiters nicht reflektiert. Der Wellenleiter - im neuwertigen Zustand - verhält sich dann wie ein unendlich langes, homogenes Kabel ohne irgendwelche Unstetigkeiten, an dessen Ende keine Signalreflexion stattfindet.

Im Laufe des Betriebs verändert sich jedoch der Tragriemen, insbesondere natürlich auch die in ihm eingebundenen Zugträger. Die permanente Zugbeanspruchung und die wiederkehrenden Biegewechsel an den Umlenkrollen verschleißen das Tragmittel. Der Verschleiß kann dabei die Zugfestigkeit des Tragmittels reduzieren und es schließlich an den Punkt sogenannten "Ablegereife" bringen.

Die mechanischen Prozesse, die zu solchen Verschleißerscheinung durch Zugkräfte und Biegewechsel führen, sind vielfältig und unterschiedlich. Durch den Verschleiß verringert sich beispielsweise der Querschnitt der einzelnen Corde in der Polymermatrix. Dies kann verursacht sein durch eine permanente Reibung (Fretting) der Filamente der Zugträger untereinander, die zu einer Form- und Querschnittsänderung der Filamente und letztlich der Zugträger führt. Auch können durch gleichzeitige Überlastungen Litzenbrüche auftreten. Darüber hinaus kann es passieren, dass die Relativbewegung der Filamente gegeneinander zu metallischem Abrieb führt.

Solche Effekte führen zu örtlich begrenzten Unstetigkeiten oder Veränderungen innerhalb des Tragriemens bzw. innerhalb der Corde des Tragriemens. Solche Unstetigkeiten oder Veränderungen ändern aber den Wellenwiderstand, d.h. die Wellenimpedanz des hier aus jeweils zwei Zugträgern bestehenden Wellenleiters und erzeugen damit Reflexionen des eingegebenen Signalimpulses.

Im Vergleich zum Neuzustand, bei dem keine Reflexionen auftreten, erhält man also bei einem merkbaren Auftreten von Reflexionen nicht nur einen deutlichen Hinweis darauf, dass solche Unstetigkeiten oder Veränderungen entstanden und vorhanden sind, sondern kann auch anhand eines vorgegebenen Katalogs ermitteln, welcher Art die Veränderungen sind und damit Rückschlüsse auf die Ablegereife des Tragriemens ziehen.

Trifft der Signalimpuls im Zugträger beispielsweise auf eine der oben genannten Form- und Querschnittsänderung der Zugträger, so erfolgt durch die geänderte Wellenimpedanz eine Teilreflexion. Aus dem Zeitpunkt des Eintreffens der Reflexion und deren Natur kann dann auf Ort und Ausmaß der Veränderung geschlossen werden. Wenn an der Störstelle/Unstetigkeit das eingespeiste impulsartige Signal teilweise reflektiert wird, kann aus der Zeitspanne zwischen der Einspeisung des Signals und dem Eintreffen der Reflexion sich bei bekannter Ausbreitungsgeschwindigkeit der Abstand der Störstelle von der Einspeisestelle ermittelt werden (Entfernung = Geschwindigkeit * Laufzeit / 2), was bei bekannter Länge der Tragriemen in einem Aufzugsystem leicht zur Lokalisierung der Unstetigkeit führt.

Die Amplituden von reflektierten Signalen sind abhängig von dem Verhältnis der aktuellen Wellenimpedanz zu der Impedanz des Widerstandsnetzwerkes welches am Zweiten Ende des Tragriemens zum Abschluss der Wellenleiter dient. Auch daraus kann vorteilhafterweise ein Maß für die Ablegereife abgeleitet werden.

Eine vorteilhafte Weiterbildung besteht darin, dass die Zugträger aus metallischen Corden ausgebildet sind und der Signalimpuls auf den ersten der jeweils als Wellenleiter geschalteten zwei Zugträger über eine elektrische bzw. galvanische Kontaktierung des jeweiligen metallischen Cords aufgegeben bzw. eingekoppelt wird. Hier wird also eine sehr einfache Art der elektrischen Kontaktierung genutzt, die ohne großen Aufwand hergestellt werden kann und keiner zusätzlicher Einrichtungen bedarf.

Eine weitere vorteilhafte Ausbildung besteht darin, dass die Zugträger aus metallischen Corden ausgebildet sind und der Signalimpuls auf den ersten der jeweils als Wellenleiter geschalteten zwei Zugträger elektromagnetisch aufgegeben bzw. eingekoppelt wird, vorzugsweise mit Hilfe einer Koppelspule des Signalgenerators auf den jeweiligen leitfähigen Cord übertragen wird. Eine solche Möglichkeit ist dann besonders vorteilhaft, wenn die Enden der Zugriemen nicht gut zugänglich und die Kopplung schwierig ist, so dass eine berührungslose Anwendung die bessere Alternative darstellt.

Eine weitere vorteilhafte Ausbildung besteht darin, dass die Zugträger aus metallischen Corden ausgebildet sind und der Signalimpuls auf den ersten der jeweils als Wellenleiter geschalteten zwei Zugträger magnetostriktiv aufgegeben bzw. eingekoppelt wird.

Dabei werden anstelle elektrischer Impulse (Strom- oder Spannung) mechanische Impulse in die Corde eingespeist. Eine Spule erzeugt lokal ein starkes Magnetfeld, das die Elementarmagnete in den Zugträgeren/Corden ausrichtet und durch Magnetostriktion zu lokaler mechanischer Spannung führt, die sich als Welle auf den Corden ausbreitet. An beiden Enden des Tragriemens befindet sich dann eine Auswerte-Einheit. Sie erzeugt ein statisches Magnetfeld, das durch die eintreffenden mechanischen Wellen gestört wird. Diese Änderung in der Magnetisierung wird dann verstärkt und ausgewertet.

Eine weitere vorteilhafte Ausbildung besteht darin, dass die anhand des Verhältnisses von eingekoppelter zu reflektierter Signalamplitude erfolgende Ermittlung der Art und Ausprägung der die Reflexion erzeugenden Stelle im Tragriemen und deren Klassifikation als eine die Ablegereife kennzeichnende Schädigung durch eine Analyse eines Musters weiterer Betriebsparametern über einen künstliche Intelligenz bereitstellenden Algorithmus erfolgt, wobei die mindestens die Häufigkeit, Höhe und Zeitdauer der Belastung des Tragriemens als Parameter in die Musteranalyse eingehen. Hierdurch entsteht ein über die Dauer der Zeit selbstlernendes System, welches auf die intrinsischen Besonderheiten des Tragmittels bzw. seiner Anwendung in einem Aufzugsystem reagieren kann und permanent die Vorhersage der Ablegereife verbessert.

Zur Durchführung des erfindungsgemäßen Verfahrens ist eine Einrichtung vorteilhaft nutzbar, bei der innerhalb eines Trag- bzw. Aufzugsystems an einem ersten Ende eines Tragriemen aus elastomerem Material mit in seiner Längsrichtung im Riemenkörper koaxial eingebetteten elektrisch leitfähigen Zugträgern bzw. Corden ein Signalgenerator sowie ein Koppelelement vorgesehen sind, mit deren Hilfe elektrische Impulse in jeweils mindestens einen von je zwei als Wellenleiter verschalteten Zugträgern des Tragmittels einkoppelbar und auskoppelbar sind. Weiterhin enthält die Einrichtung eine mit Signalgenerator und Koppelelement kommunizierende und die Signalimpulse und deren Reflexionen im jeweiligen Wellenleiter auswertende Elektronik (Auswerteelektronik). An einem bzw. an dem zweiten Ende des Tragriemens ist jeder Zugträger über eine Netzwerksanordnung von mehreren Widerständen (Einzelimpedanzen) mit einem ihm jeweils zu einem Wellenleiter zugeordneten weiteren Zugträger über einen der Widerstände verbindbar und so der Wellenleiter abschließbar.

Vorteilhafterweise lässt sich eine solche Einrichtung in einem Aufzugsystem mit einem oder mehreren Tragriemen mit in ihrer Längsrichtung im Riemenkörper koaxial eingebetteten elektrisch leitfähigen Zugträgern verwenden. Da die Tragriemen üblicherweise zwei festgelegte Enden besitzen, kann in einer einfachsten Ausführung mindestens ein Signalgenerator sowie ein Koppelelement an einem ersten feststehenden Ende mindestens eines Tragriemens und eine Netzwerksanordnung von mehreren Widerständen am jeweils feststehenden zweiten Ende des Tragriemens angeordnet sein Anhand eines Ausführungsbeispiels soll die Erfindung näher erläutert werden.

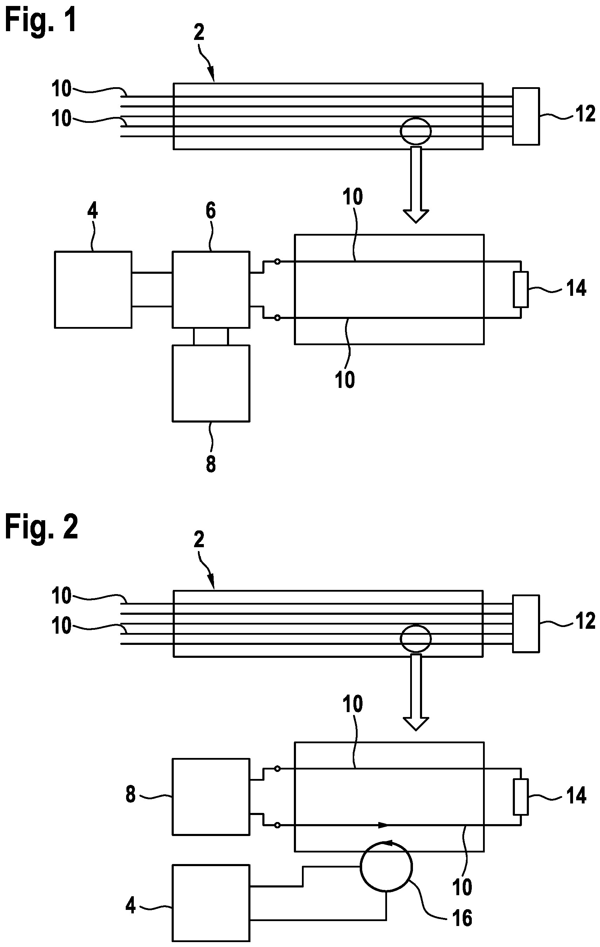

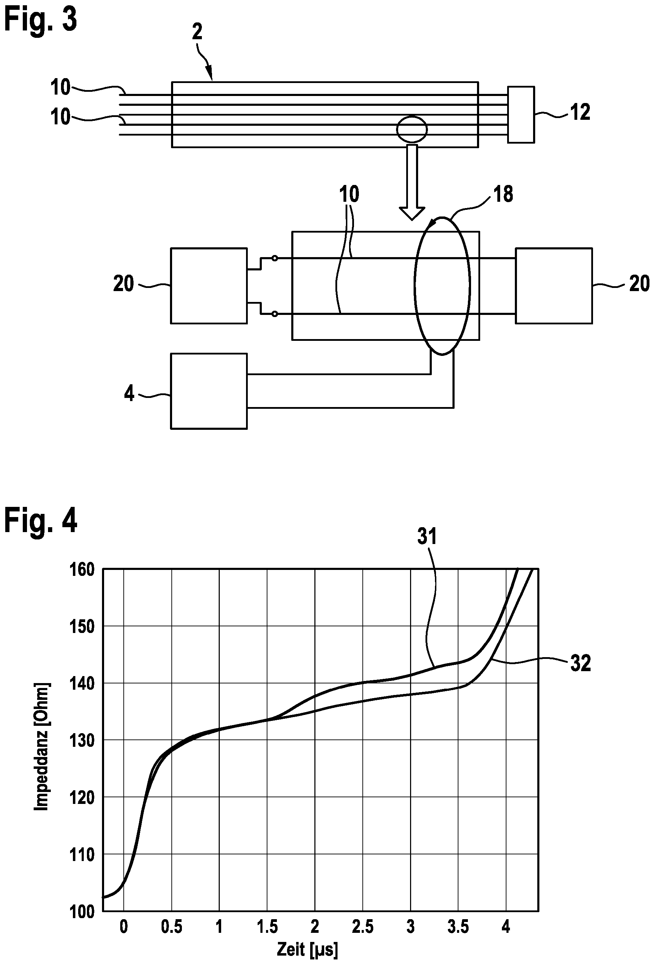

Die Fig. 1 bis 3 zeigen lediglich prinzipiell eine Einrichtung, die für die Durchführung des erfindungsgemäßen Verfahrens geeignet ist. Figur 4 zeigt in einem Diagramm die ortsabhängige Eingangsimpedanz des jeweiligen Wellenleiters bei der Messung durch die Auswerteeinheit.

Die Fig. 1 zeigt in ihrem oberen Teil eine Prinzipskizze, die eine Gesamtansicht des Tragriemens 2 mit den Zugträgern 10 und einem Bauteil 12 darstellt, welches ein aus mehreren Einzelimpedanzen aufgebauten Netzwerks an einem zweiten Ende des Tragriemens beinhaltet. Im unteren Teil zeigt die Figur 1 einen Ausschnitt aus dem oberen Teil, nämlich einen ersten und ein zweiten Zugträger 10 des Tragriemens 2, wobei die beiden Zugträger als Wellenleiter innerhalb einer Mess-Elektronik verschaltet sind, die einen Signalgenerator 4, ein Koppelelement 6 und eine Auswerte-Elektronik 8 aufweist. Das erfindungsgemäße System arbeitet nach dem Verfahren der Time-Domain-Reflectometry (TDR). An dem ersten Ende des Tragriemens 2 befinden sich, wie gesagt, der Signalgenerator 4, das Koppelelement 6 zur galvanisch/elektrischen Kopplung des Signalimpulses über elektrische Kontaktierung und die Auswerte-Elektronik 8. Die beiden elektrisch leitfähige Corde 10 des Tragmittels 2 bilden den Wellenleiter, in den der Signalgenerator 4 über das Koppelelement 6 elektrische Impulse einspeist. Diese Impulse breiten sich entlang des Wellenleiters aus.

An dem zweiten Ende des Tragmittels 2 befindet sich ein Netzwerk 12 aus Widerständen, das alle gebildeten Wellenleiter mit der Wellenimpedanz eines intakten, fabrikneuen Tragmittels so abschließen kann, so dass die elektrischen Impulse nicht reflektiert werden. Die zwei Zugträger 10 sind also dabei über einen Widerstand 14 abgeschlossen bzw. miteinander verbunden, dessen Widerstandswert Z₀ gleich groß ist wie der Leitungswellenwiderstand (Wellenimpedanz), der zum Zeitpunkt der Inbetriebnahme eines neu aufgelegten Tragriemens bei der Verschaltung zweier Zugträger zu einem Wellenleiters vorhanden ist. Das Netzwerk besteht dabei aus mehreren schaltbaren Einzelwiderständen/Einzelimpedanzen.

Ändert sich die Wellenimpedanz der Zugträger des Tragriemens 2 z. B. durch die Verringerung ihres tragenden Querschnittes infolge von Reibung der einzelnen Filamente gegeneinander und dem dadurch hervorgerufenen Verschleiß (Fretting), werden die elektrischen Signalimpulse an dem zweiten Ende des Tragmittels 2 reflektiert. Die Amplituden der reflektierten Signale sind abhängig von dem Verhältnis der aktuellen Wellenimpedanz zu der Impedanz des Abschlussnetzwerkes, so dass daraus ein Maß für die Ablegereife abgeleitet werden kann z. B. als ortsabhängige Eingangsimpedanz, wie in der Kurve in Fig. 4 dargestellt. Die untere Kurve 30 in der Figur 4 repräsentiert dabei ein ungeschädigtes Tragmittel, während die ober Kurve 31 die eines geschädigten Tragmittels ist.

Örtlich begrenzte Unstetigkeiten des Tragmittels, wie sie z. B. durch Veränderungen der Filamente infolge der Dauerbelastung entstehen, führen zu einer Veränderung der lokalen Impedanz gegenüber der Wellenimpedanz des Tragmittels. An der Stelle der Unstetigkeit wird das eingespeiste impulsartige Signal teilweise reflektiert. Aus der Zeitspanne zwischen der Einspeisung des Signals und dem Eintreffen der Reflexion lässt sich bei bekannter Ausbreitungsgeschwindigkeit der Abstand der Störstelle von der Einspeisestelle ermitteln.

Fig. 2 entspricht in Ihrem oberen Teil der Fig. 1 und zeigt in Ihrem unteren Teil einen Ausschnitt aus dem oberen Teil, nämlich den Signalgenerator 4 mit einem als Koppelspule 16 ausgebildeten Koppelelement zur elektromagnetischen Kopplung des Signalimpulses sowie die Auswerte-Elektronik 8. Die beiden elektrisch leitfähige Corde 10 des Tragmittels 2 bilden hier auch wieder den Wellenleiter, in den der Signalgenerator 4 über die Koppelspule 16 Signalimpulse einspeist.

Fig. 3 entspricht in Ihrem oberen Teil auch wieder der Fig. 1 und zeigt in Ihrem unteren Teil daraus einen Ausschnitt mit u.a. dem Signalgenerator 4. In dieser Ausführung werden mechanische Impulse in die metallischen Zugträger/Corde 10 eingespeist. Eine Spule 18 erzeugt dazu lokal ein starkes Magnetfeld, das die Elementarmagnete in den Corden 10 ausrichtet und durch Magnetostriktion zu lokaler mechanischer Spannung führt, die sich als Welle auf / bzw. in den Corden ausbreitet. An beiden Enden des der Corde bzw. des Tragriemens befindet sich je eine Auswerte-Einheit 20. Sie erzeugt ein statisches Magnetfeld, das durch die eintreffenenden mechanischen Wellen gestört wird. Diese Änderung in der Magnetisierung wird verstärkt und ausgewertet.

### Bezugszeichenliste

### (Teil der Beschreibung)

- 2: Tragmittel bzw. Tragriemen
- 4: Signalgenerator
- 6: Koppelelement
- 8: Auswerteelektronik
- 10: Zugträger/Corde
- 12: aus mehreren Einzelimpedanzen aufgebautes Netzwerk
- 14: Widerstand Z₀ / Abschlusswiderstand
- 16: Koppelspule
- 18: Spule
- 20: Auswerte-Einheit
- 30: Kurve eines ungeschädigten Tragmittels
- 31: Kurve eines geschädigten Tragmittels

## Patentansprüche

1. Verfahren zur Detektion eines Abnutzungszustands eines Tragriemens (2) aus elastomerem Material innerhalb eines Trag- bzw. Aufzugsystems mit Hilfe der Zeitbereichsreflektometrie, wobei die Tragriemen in ihrer Längsrichtung im Riemenkörper koaxial eingebettete elektrisch leitfähige Zugträger (10) aufweisen, die als aus mehreren Filamenten getwistete oder geschlagene Corde ausgebildet sind, **gekennzeichnet durch** folgende Merkmale:
- jeweils ein erster und ein zweiter Zugträger (10) des Tragriemens (2) werden als Wellenleiter innerhalb einer Mess-Elektronik verschaltet, wobei
- die jeweils als Wellenleiter geschalteten zwei Zugträger mit Hilfe eines aus mehreren Einzelimpedanzen aufgebauten Netzwerks (12) an einem zweiten Ende des Tragriemens über einen Widerstand abgeschlossen bzw. miteinander verbunden werden, dessen Widerstandswert Z₀ gleich groß ist wie der Leitungswellenwiderstand (Wellenimpedanz), der zum Zeitpunkt der Inbetriebnahme eines neu aufgelegten Tragriemens bei der Verschaltung zweier Zugträger zu einem Wellenleiters vorhanden ist,
- an einem ersten Ende des Tragriemens wird durch einen Signalgenerator (4) ein Signalimpuls auf den ersten der jeweils als Wellenleiter geschalteten zwei Zugträger aufgegeben bzw. eingekoppelt,
- über eine Auswerteelektronik (8, 20), vorzugsweise am ersten Ende des Tragriemens, wird dann ermittelt, ob nach Einkopplung des Signalimpulses eine Reflexion des Signalimpulses vorhanden ist und welche Amplituden und Zeitverläufe die reflektierten Signalimpulse aufweisen,
wonach bei einer detektierten Reflexion eines Signalimpulses anhand der Signallaufzeiten der Abstand der die Reflexion erzeugenden Stelle von der Einkoppelstelle am ersten Ende berechnet und die Ausprägung der die Reflexion erzeugenden Stelle im Tragriemen anhand eines vorgegebenen Katalogs ermittelt und abhängig davon auf die Ablegereife des Tragriemens (2) geschlossen wird.

2. Verfahren nach Anspruch 1, bei dem die Art und Ausprägung der die Reflexion erzeugenden Stelle im Tragriemen anhand des Verhältnisses von eingekoppelter zu reflektierter Signalamplitude ermittelt wird.

3. Verfahren nach Anspruch 1 oder 2, bei dem die Zugträger aus metallischen Corden ausgebildet sind und der Signalimpuls auf den ersten der jeweils als Wellenleiter geschalteten zwei Zugträger über eine elektrische bzw. galvanische Kontaktierung des jeweiligen metallischen Cords aufgegeben bzw. eingekoppelt wird.

4. Verfahren nach Anspruch 1 oder 2, bei dem die Zugträger aus metallischen Corden ausgebildet sind und der Signalimpuls auf den ersten der jeweils als Wellenleiter geschalteten zwei Zugträger elektromagnetisch aufgegeben bzw. eingekoppelt wird, vorzugsweise mit Hilfe einer Koppelspule des Signalgenerators auf den jeweiligen leitfähigen Cord übertragen wird.

5. Verfahren nach Anspruch 1 oder 2, bei dem die Zugträger aus metallischen Corden ausgebildet sind und der Signalimpuls auf den ersten der jeweils als Wellenleiter geschalteten zwei Zugträger magnetostriktiv aufgegeben bzw. eingekoppelt wird.

6. Verfahren nach einem der Ansprüche 1 bis 5, bei dem die anhand des Verhältnisses von eingekoppelter zu reflektierter Signalamplitude erfolgende Ermittlung der Art und Ausprägung der die Reflexion erzeugenden Stelle im Tragriemen und deren Klassifikation als eine die Ablegereife kennzeichnende Schädigung durch eine Analyse eines Musters weiterer Betriebsparametern über künstliche Intelligenz erfolgt, wobei die mindestens die Häufigkeit, Höhe und Zeitdauer der Belastung des Tragriemens als Parameter in die Musteranalyse eingehen.

7. Einrichtung zur Durchführung des Verfahrens nach Anspruch 1 bis 6, **dadurch gekennzeichnet, dass** innerhalb eines Trag- bzw. Aufzugsystems an einem ersten Ende eines Tragriemen (2) aus elastomerem Material mit in seiner Längsrichtung im Riemenkörper koaxial eingebetteten elektrisch leitfähigen Zugträgern (10) bzw. Corden ein Signalgenerator (4) sowie ein Koppelelement (6, 16, 18) vorgesehen sind, mit deren Hilfe vorzugsweise elektrische/elektromagnetische Signalimpulse in jeweils mindestens einen von je zwei als Wellenleiter verschalteten Zugträgern (10) des Tragmittels einkoppelbar und/oder auskoppelbar sind, dass eine mit Signalgenerator (4) und Koppelelement (6) kommunizierende und die Signalimpulse und deren Reflexionen im jeweiligen Wellenleiter auswertende Auswerteelektronik (8, 20) vorgesehen ist, dass an einem zweiten Ende des Tragriemens jeder Zugträger über eine Netzwerksanordnung (12) von mehreren Widerständen mit einem ihm jeweils zu einem Wellenleiter zugeordneten weiteren Zugträger über einen der Widerstände (14) verbindbar und so der Wellenleiter abschließbar ist.

8. Aufzugsystem mit einem oder mehreren Tragriemen (2) mit in ihrer Längsrichtung im Riemenkörper koaxial eingebetteten elektrisch leitfähigen Zugträgern (10), aufweisend eine Einrichtung nach Anspruch 7, bei dem mindestens ein Signalgenerator (4) sowie ein Koppelelement (6, 16, 18) an einem ersten feststehenden Ende mindestens eines Tragriemens und eine Netzwerksanordnung (12) von mehreren Widerständen am jeweils feststehenden zweiten Ende des Tragriemens angeordnet sind.
